# EUROPEAN PATENT APPLICATION

(11) **EP 4 257 977 A1**
(43) Date of publication of application: **11.10.2023**
(21) Application number: 21903323.0
(22) Date of filing: 03.12.2021
(51) Int. Cl.: G01N 33/53, A61K 45/00, A61P 13/12, A61P 37/06, A61P 43/00, C07K 16/18, C12N 15/11, G01N 33/15, G01N 33/50

(54) **AUTOANTIBODIES RELATED TO IGA NEPHROPATHY**

(30) Priority: 07.12.2020 JP 2020202925
(71) Applicant: Juntendo Educational Foundation, Tokyo 113-8421 (JP); TOKYO UNIVERSITY OF SCIENCE FOUNDATION, Tokyo 162-8601 (JP)
(72) Inventor: SUZUKI, Yusuke, Tokyo 113-8421 (JP); NIHEI, Yoshihito, Tokyo 113-8421 (JP); KITAMURA, Daisuke, Tokyo 162-8601 (JP)
(74) Representative: Plougmann Vingtoft a/s
(86) International application number: PCT/JP2021/044409
(87) International publication number: WO 2022/124219

(57) **Abstract**

Novel factors which are involved in the mechanism of the onset of IgA nephropathy are explored, and diagnostic, preventive or therapeutic agents for this disease are provided. A method for measuring autoantibodies related to IgA nephropathy, the method comprising measuring the amount of anti-spectrin β IgA antibodies in a sample derived from a living organism.

## Description

### Technical Field

The present invention relates to an autoantibody related to IgA nephropathy and use thereof.

### Background Art

IgA nephropathy is the most common primary glomerulonephritis in the world, especially in Japan and other Asian countries. If this disease is left untreated, approximately 30 to 40% of cases will lead to end-stage renal failure with poor prognosis. This disease is designated as an intractable disease (designated intractable disease No. 66).

IgA nephropathy is defined as chronic glomerulonephritis with proliferative changes in glomerular mesangial cells and matrix, and with deposits of mainly IgA in the mesangial region. In recent years, it has been proved that IgA deposited in the renal glomeruli in IgA nephropathy is IgA1 with undergalactosylated O-linked glycans in its hinge region (galactose-deficient IgA1: Gd-IgA1). Gd-IgA1 shows extremely high disease specificity. Therefore, an increase of blood Gd-IgA1 is considered to be the first hit for the onset of this disease.

On the other hand, it has been found that Gd-IgA1 is also present in blood relatives of IgA nephropathy patients who have not developed nephritis and from healthy individuals, suggesting that the increase of blood Gd-IgA1 alone is insufficient for the onset of the disease. The possibility that Gd-IgA1 is not directly involved in the onset of this disease has also been discussed (Non-Patent Literatures 1 and 2).

### Citation List

### Non-Patent Literature

Non-Patent Literature 1: B Wehbi, et al. J Am Soc Nephrol 2019; 30: 1238-1249
Non-Patent Literature 2: A Takahata, et al. J Am Soc Nephrol 2020; 31 (9), 2013-2024

### Summary of Invention

### Technical Problem

As described above, factors which are directly involved in the development of IgA nephropathy have not yet been clarified, and is a need to explore new factors which are involved in the mechanism of the onset of IgA nephropathy.

Therefore, an object of the present invention is to explore novel factors which are involved in the mechanism of the onset of IgA nephropathy, and to provide diagnostic, preventive and therapeutic agents for this disease.

### Solution to Problem

Accordingly, the present inventors conducted various investigations on the hypothesis that IgA-type autoantibodies against renal glomerular components may be involved in the mechanism of the onset of IgA nephropathy. First, the present inventors confirmed the presence of IgA autoantibodies against renal glomeruli in the serum of model mice of spontaneous IgA nephropathy (gddY mice) and human serum. Next, proteins recognized by the gddY mouse serum IgA antibodies were isolated from renal glomerular proteins and analyzed, and autoantigen candidate proteins recognized by the autoantibodies were identified. Further, recombinant proteins of these candidate proteins were produced, and it was examined whether they were recognized by the gddY mouse serum. As a result, it was found that most of the gddY mice had, in their serum, IgA autoantibodies against spectrin β present in the mesangial cells. It was also confirmed that the IgA autoantibodies against spectrin β were also present in the serum of most of the patients with IgA nephropathy. Further, it was confirmed that spectrin in the cytoplasm was presented on the cell surface by MHC class II molecules.

Thus, the present inventors clarified that the anti-spectrin β IgA antibodies are present in the serum of IgA nephropathy patients, and that the antibodies are deposited in mesangial cells in an MHC-dependent manner. That is, it was found that IgA nephropathy can be considered to be an autoimmune disease because the patients have tissue-specific autoantibodies. Accordingly, it was found that IgA nephropathy can be diagnosed by measuring the concentration of anti-spectrin β IgA antibody in biological samples, such as serum; that drugs which inhibit the activity or production of anti-spectrin β IgA antibodies are useful as preventive or therapeutic agents for IgA nephropathy; and that preventive or therapeutic agents for IgA nephropathy can be screened by screening drugs which inhibit the activity or production of anti-spectrin β IgA antibodies.

Specifically, the present invention provides the following inventios [1] to [8].
[1] A method for measuring autoantibodies related to IgA nephropathy, the method comprising measuring an amount of anti-spectrin β IgA antibodies in a sample derived from a living organism.
[2] A diagnostic agent for IgA nephropathy comprising an anti-spectrin β IgA antibody measurement reagent.
[3] The diagnostic agent for IgA nephropathy according to [2], wherein the anti-spectrin β IgA antibody measurement reagent is an immunological measurement reagent comprising spectrin β.
[4] A method for screening a preventive or therapeutic agent for IgA nephropathy, the method comprising screening a drug which inhibits activity or production of anti-spectrin β IgA antibodies.
[5] A preventive or therapeutic agent for IgA nephropathy comprising a drug which inhibits activity or production of anti-spectrin β IgA antibodies as an active ingredient.
[6] A drug which inhibits activity or production of anti-spectrin β IgA antibodies for use in prevention or treatment of IgA nephropathy.
[7] Use of a drug which inhibits activity or production of anti-spectrin β IgA antibodies for producing a preventive or therapeutic agent for IgA nephropathy.
[8] A method for preventing or treating IgA nephropathy, the method comprising administering an effective amount of a drug which inhibits activity or production of anti-spectrin β IgA antibodies.

### Advantageous Effects of Invention

The present invention has clarified that IgA nephropathy is an autoimmune disease with tissue-specific autoantibodies, and has revealed that the target autoantibodies are anti-spectrin β IgA antibodies. In addition, the present invention provides a new diagnostic technique for IgA nephropathy, a preventive or therapeutic agent for IgA nephropathy, and a method for screening a preventive or therapeutic agent for IgA nephropathy.

### Brief Description of Drawings

[Figure 1A] Figure 1A shows immunofluorescent staining using kidney sections of activation-induced cytidine deaminase (AID) knockout mice deficient in endogenous IgA antibodies. The serum of 16W Balb/c (left) or gddY (right) mice was used as the primary antibody, and PE-labeled anti-IgA antibody was used as the secondary antibody. The green circles represent glomerular regions.
[Figure 1B] Figure 1B shows the immunoblot results of lysates of glomeruli of gddY mice blotted with serum IgA derived from 12W BALB/c or 8W gddY mice (n=3). Each lane shows the serum of each mouse.
[Figure 1C] Figure 1C shows the immunoblot result of lysates of mouse primary mesangial cells (MCs) blotted with serum IgA derived from 12W Balb/c (left) or 8W gddY (right) mice (N=3). Each lane shows the serum of each mouse.
[Figure 1D] Figure 1D shows the reactivity of serum IgA derived from 8W-16W Balb/c or 8-16W gddY mice against p250+.
[Figure 2A] Figure 2A shows the immunoblot results using fetal kidney cells (HEK) 293T cells, infected with a mock vector (M) or a FLAG-tagged sptbn1A expression vector (1A). The serum of Balb/c (N=4) or gddY (N=4) was used as the primary antibody, and anti-IgA antibody was used as the secondary antibody. The overexpression of FLAG-tagged sptbn1A was confirmed with anti-FLAG antibody (lower figure).
[Figure 2B] Figure 2B shows the immunoblot results using HEK293T cells infected with a mock vector (M) or a FLAG-tagged sptbn1B expression vector (1B). The serum of Balb/c (N=4) or gddY (N=4) was used as the primary antibody, and anti-IgA antibody was used as the secondary antibody. The overexpression of FLAG-tagged sptbn1B was confirmed with anti-FLAG antibody (lower figure).
[Figure 2C] Figure 2C shows the immunoblot results using HEK293T cells infected with a mock vector (M) or a FLAG-tagged sptbn1C expression vector (1C). The serum of Balb/c (N=4) or gddY (N=4) was used as the primary antibody, and anti-IgA antibody was used as the secondary antibody. The red arrow indicates the presence of anti-Sptbn1C IgA antibody in serum derived from gddY mouse. The overexpression of FLAG-tagged sptbn1C was confirmed with anti-FLAG antibody (lower figure).
[Figure 2D] Figure 2D shows the reactivity of serum IgA derived from 12-16W Balb/c (left) and 8-16W gddY (right) mice against sptbn1C.
[Figure 3A] Figure 3A shows the immunoblot results of primary human MC lysates using the serum of healthy subjects (healthy control: HC) or IgA nephropathy (IgAN) patients. The red arrow indicates the presence of IgA autoantibody against p250+ in the serum of the IgA nephropathy patients. Numbers #1 to #5 indicate the number of IDs for each individual.
[Figure 3B] Figure 3B shows the reactivity of serum IgA derived from HC or IgA nephropathy patients against p250+.
[Figure 3C] Figure 3C shows the immunoblot results using HEK293T cells infected with a mock vector (M) or a FLAG-tagged full-length SPTBN1 expression vector (S). The serum of HC, or patients with other renal disease (disease control: DC) or IgA nephropathy (IgAN) was used as the primary antibody, and anti-IgA antibody was used as the secondary antibody (upper panel). The red arrow indicates the presence of anti-SPTBN1 IgA1 antibody in the serum of the IgA nephropathy patients. The overexpression of FLAG-tagged SPTBN1 was confirmed with anti-FLAG antibody (lower figure).
[Figure 3D] Figure 3D shows the reactivity of serum IgA1 against SPTBN1 of the serum of healthy subjects, other nephritis patients, or IgA nephropathy patients.
[Figure 4A] Figure 4A shows the results of flow cytometry analysis of the expression of CD138 and IgA in mononuclear cells isolated from the kidney of the indicated mice (intracellular staining). The numbers represented adjacent to the outline regions indicate the percentage of IgA+ plasma cells (PCs) in live cells. The mice used were gddY (8-week-old), Balb/c (8-week-old), NZB/w F1 (24-week-old), and Fas^{lpr/lpr} (24-week-old) mice.
[Figure 4B] The expression of Ki67 in the following cell groups was analyzed by flow cytometry. IgA+ PCs infiltrating the kidney of gddY (blue), IgA+ PCs present in the small intestine of C57BL/6 mice (red), germinal center B cells in the spleen of C57BL/6 mice immunized with hapten 4-hydroxy-3-nitrophenylacetyl (NP)-chicken gamma globulin and alum 6 days before analysis (green), and naive B cells in the spleen of C57BL/6 mice (grey).
[Figure 4C] Figure 4C shows the frequency of IgA+ PCs in live cells in the kidney of Balb/c (blue), NZB/w F1 (green), Fas^{lpr/lpr} (purple), or gddY (red). Quantification results of Figure 4A. Each point represents one mouse. In Figure 4C, ** p<0.01, *** p<0.001, **** p<0.0001 (one-way analysis of variance (ANOVA) with multiple comparison test.
[Figure 4D] Figure 4D shows the number of infiltrating IgA+PC cells per kidney in the mice of Figure 4C. Each point represents one mouse. In Figure 4D, ** p<0.01, *** p<0.001, **** p<0.0001 (one-way analysis of variance (ANOVA) with multiple comparison test.
[Figure 4E] Figure 4E shows the frequency of IgA+ PCs in live cells derived from gddY mice of each age. Each point represents one mouse.
[Figure 4F] Figure 4F shows the immunofluorescent staining of the kidney of gddY mice. IgA was stained with red and CD138 was stained with blue.
[Figure 4G] Figure 4G shows the results of analyzing the frequency of IgG+ (blue) or IgA+ (red) cells among PCs infiltrating the kidney of gddY mice. In Figure 4G, **** p<0.0001 (Student's t-test).
[Figure 5A] Figure 5A shows the results of immunohistochemical staining of a kidney biopsy section from an IgA nephropathy patient. The brown arrows indicate IgA+ cells.
[Figure 5B] Figure 5B shows a plot of serum creatinine (S-creatinine) levels of each patient against the number of plasma cells per glomerulus in each biopsy of the corresponding patient.
[Figure 5C] Figure 5C shows a plot of proteinuria levels of each patient against the number of plasma cells per glomerulus in each biopsy of the corresponding patient.
[Figure 6A] Figure 6A shows a method for culturing IgA+ plasmablasts (PBs) infiltrating the kidney of gddY mice.
[Figure 6B] Figure 6B shows immunofluorescent staining of kidney sections of AID knockout mice. 12W Balb/c mouse-derived serum IgA or a culture supernatant of IgA+ PBs infiltrating the kidney of gddY mice was used as the primary antibody, and PE-labeled anti-IgA antibody was used as the secondary antibody. The green circles represent glomerular regions.
[Figure 6C] Figure 6C shows the immunoblot results of glomerular lysates using a culture medium alone (Control) or a culture supernatant of IgA+ PBs of the kidney of gddY mice (kidney) as the primary antibody, and anti-IgA antibody as the secondary antibody.

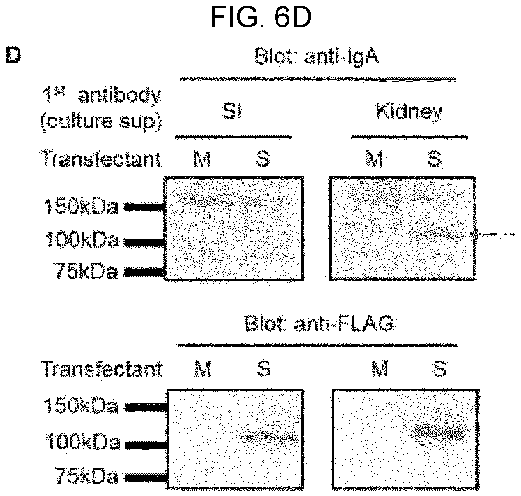

[Figure 6D] Figure 6D shows the immunoblot using HEK293T cells infected with a mock vector (M) or a FLAG-tagged sptbn1C expression vector (S). A culture supernatant of IgA+ PCs present in the small intestinal lamina propria of gddY (SI) or IgA+ PCs infiltrating the kidney (kidney) was used as the primary antibody, and anti-IgA antibody was used as the secondary antibody (upper figure). The red arrow indicates the presence of anti-Sptbn1C IgA antibody in the culture supernatant of IgA+ PBs infiltrating the kidney of gddY. The overexpression of FLAG-tagged sptbn1C was confirmed with anti-FLAG antibody (lower figure).
[Figure 6E] Recombinant IGA antibodies were generated from IgA+ PBs infiltrating the kidney of gddY mice. The binding ability of each antibody to mouse MCs was analyzed by flow cytometry (intracellular staining). As a control, IgA antibody with affinity for NP was used (#NP). The binding ability of clone #5 (negative), #9 (slightly positive) and #11 (positive) to MCs was analyzed. MCs stained only with the secondary antibody (anti-IgA) are shown in gray.
[Figure 6F] Figure 6F shows the binding ability of recombinant IgA antibodies to MCs by flow cytometry.
[Figure 6G] The binding ability of #NP or clone #9 to sptbn1C was analyzed by Western blot (WB). Proteins immunoprecipitated with anti-FLAG antibody from HEK293T cells infected with a mock vector (M) or a FLAG-tagged sptbn1C expression vector (S) were immunoblotted using recombinant antibodies (upper figure). #NP or clone #9 was used as the primary antibody, and anti-IgA antibody was used as the secondary antibody. The red arrow indicates that clone #9 recognizes sptbn1C. The expression of FLAG-tagged sptbn1C was confirmed with anti-FLAG antibody (lower figure).
[Figure 6H] Figure 6H shows the binding ability of recombinant antibodies to sptbn1C, generated from IgA + PBs derived from the kidney of gddy mice.

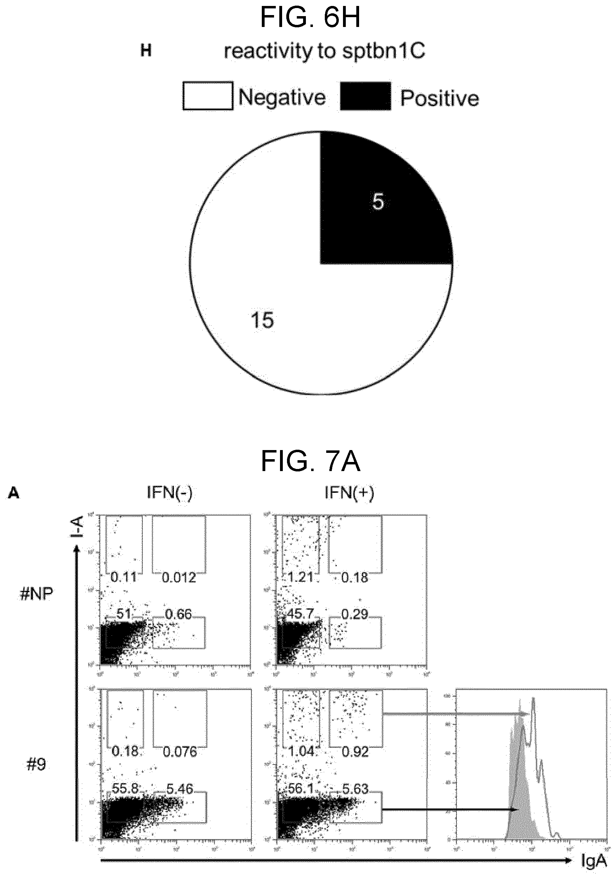

[Figure 7A] The binding ability of #NP or clone #9 to MCs cultured in the presence or absence of IFN-γ was analyzed by flow cytometry. MCs were cultured in the presence or absence of IFN-γ (50 ng/mL) for 48 hours, and cell surface staining was performed using anti-I-A(H2-S) antibody and recombinant antibody as the primary antibodies, and anti-IgA antibody as the secondary antibody. The numbers represented adjacent to the outline regions indicate the frequency of I-A-/IgA-, I-A-/IgA+, I-A+/IgA-, or I-A+/IgA+ cells between live cells. The histograms show the binding ability of clone #9 to I-A-non-expressing MCs (grey) and I-A-expressing MCs (red).
[Figure 7B] A T7-tagged sptbn1C expression vector was infected into human HEK293T cells expressing C57BL/6- or gddY-derived I-A (C57BL/6 I-A HEK and gddY I-A HEK). Analysis of the surface expression of T7 in each cell by flow cytometry is shown.
[Figure 7C] A mock vector (M) or a T7-tagged sptbn1C expression vector (S) was infected into C57BL/6 I-A HEK or gddY I-A HEK. I-A β-chains (FLAG-tagged β-chains) were immunoprecipitated from infected cell lysates using anti-FLAG antibody, and the co-precipitated protein was immunoblotted with anti-T7 antibody (top). An immunoblot of sptbn1C in the whole cell lysate (WCL) with anti-T7 antibody (middle), and an immunoblot of I-A β-chains with anti-FLAG antibody in the immunoprecipitated protein (bottom) are shown.
[Figure 8] Figure 8 shows gating diagrams of the flow cytometry of Figure 4B.
[Figure 9] Figure 9 shows gating diagrams of IgA+ PCs (CD138+ B220 low) infiltrating the kidney of gddY mice. Of plasma cells infiltrating the kidney of gddY (upper figure) or NZB/w F1 mice (lower figure), the frequency of IgA+ or IgG+ cells was analyzed by flow cytometry.
[Figure 10] Figure 10 shows the number of somatic hypermutations in the heavy and light chain variable regions of recombinant antibodies produced from IgA PBs infiltrating the kidney of gddY mice.
[Figure 11] The surface expression of I-A on mouse MCs cultured in the presence of INF-γ was analyzed by flow cytometry. Primary MCs were cultured in the presence or absence of IFN-γ, and surface-stained with PE-labeled anti-I-A antibody or isotype control antibody after 48 hours.
[Figure 12] The expression of I-A on the surface of C57BL/6 I-A HEK and gddY I-A HEK was confirmed by flow cytometry. HEK 293T cells without expression of I-A (control HEK) were shown in gray.

### Description of Embodiments

The characteristic of the present invention is that the present inventors clarified that IgA nephropathy is an autoimmune disease with tissue-specific autoantibodies, and found that the autoantibodies are anti-spectrin β IgA antibodies.

Spectrin as mentioned herein is a major component which constitutes the net structure of protein covering the plasma membrane surface of cells, such as red blood cells of vertebrates, and is a high-molecular-weight heterodimer composed of two types of subunits, α and β. Spectrin is present inside many types of cell membranes, acts as a scaffold which maintains the shape of cells, and plays an important role in maintaining the structure of cell membranes.

However, it was completely unknown that spectrin can serve as an autoantigen for autoantibodies, and that anti-spectrin β IgA antibodies are autoantibodies involved in IgA nephropathy.

There are five types (I to V) of spectrin β. Preferred in the present invention are antibodies which recognize spectrin β-II (β-II spectrin, Spectrin β, NonErythrocytic 1), i.e., anti-spectrin β-II IgA antibodies.

The method for measuring autoantibodies related to IgA nephropathy according to the present invention is characterized by measuring the amount of anti-spectrin β IgA antibodies in a sample derived from a living organism.

Examples of samples derived from living organisms to be measured include serum, plasma, kidney biopsy samples, lymph, and the like. Preferred among these are serum, plasma, and kidney biopsy samples, and particularly preferred is serum.

Examples of living organisms to be measured include animals including humans, and humans are preferred. The living organisms may be healthy one or one with suspected IgA nephropathy; however, humans with suspected IgA nephropathy are preferred.

The means for measuring the amount of anti-spectrin β IgA antibodies is preferably an immunoassay using a reagent containing spectrin β, which is an autoantigen.

The immunoassay may be any known immunological measurement method, and examples thereof include radioimmunoassay (RIA), enzyme immunoassay (EIA or ELISA), fluoroimmunoassay (FIA), indirect fluorescence assay, luminescent immunoassay, physicochemical assays (TIA, LAPIA, and PCIA), Western blotting, and the like; preferred is ELISA.

ELISA is a method of reacting an antigen immobilized on a solid phase and an antibody, further reacting the antibody binding to the antigen with a secondary antibody labeled with an enzyme, such as peroxidase or alkaline phosphatase, and then measuring the enzyme label by an appropriate process. Examples thereof include a competitive method and a sandwich method, and preferred of these is a sandwich method (solid phase sandwich method).

The solid phase sandwich method is performed, for example, as follows. Specifically, spectrin β, which is an antigen, is first immobilized on a solid phase, and a specimen as a test sample is added thereto. As a result, an antigen-antibody reaction occurs between the solid-phase antigen and antibodies in the sample, and anti-spectrin β IgA antibodies present in the specimen bind to the solid-phase antigen. Next, the bound antibodies are detected with an antibody detection reagent to measure the anti-spectrin β IgA antibodies present in the sample.

In the above method, the target anti-spectrin β IgA antibodies present in the test sample can also be detected and measured by immobilizing the antibody detection reagent on a solid phase to thereby capture antibodies in the specimen, then adding antigen spectrin β, allowing it to bind to anti-spectrin β IgA antibodies among the captured antibodies, and further allowing labeled antibodies specific to the antigen to bind thereto.

Selection of various means and modification thereof in these measurement methods are well known to those skilled in the art and are not particularly limited in the present invention. Any of these methods can be employed [see, for example, "Rinsho Kensa-ho Teiyo (Clinical Examination Handbook)", Kanehara & Co., Ltd., 1995] .

For example, as the solid phase used in the above solid-phase method, generally used insoluble and inactive carriers can be widely used. Examples thereof include sticks, beads, microplates, test tubes, and the like made of various materials, such as glass, cellulose powder, Sephadex, Sepharose, polystyrene, filter paper, carboxymethylcellulose, ion exchange resin, dextran, plastic films, plastic tubes, nylon, glass beads, silk, polyamine-methyl vinyl ether-maleic acid copolymers, amino acid copolymers, and ethylene-maleic acid copolymers.

The method for immobilizing antigens or antibodies is also not particularly limited, and either physical bonding or chemical bonding can be used. Typical examples thereof include methods using chemical reactions, such as covalent bonding methods, including diazo method, peptide method (e.g., acid amide derivative method, carboxyl chloride resin method, carbodiimide resin method, maleic anhydride derivative method, isocyanate derivative method, cyanogen bromide-activated polysaccharide method, cellulose carbonate derivative method, and method using a condensed reagent), and alkylation method, carrier-bonding methods using crosslinking reagents (using glutaraldehyde, hexamethylene isocyanate, and the like as crosslinking reagents), and carrier-bonding methods by Ugi reactions; ion-binding methods using carriers, such as ion exchange resin; physical adsorption methods using porous glass, such as glass beads, as a carrier; and the like.

The labeling agent in each measurement system is also not particularly limited, and conventionally known labeling agents can be used. Specific examples thereof include various radioactive isotopes conventionally used in immunoassays, enzymes such as alkaline phosphatase (ALP) and peroxidase (POX), fluorescent substances such as fluorescein isothiocyanate (FITC) and tetramethylrhodamine isothiocyanate (RITC), and others, including 1N-(2,2,6,6-tetramethyl-1-oxyl-4-piperidinyl)-5N-(aspartate)-2,4-dinitrobenzene (TOPA).

Examples of enzyme labeling substances for enzyme labeling include, in addition to the examples mentioned above, microperoxidase, chymotrypsinogen, procarboxypeptidase, glyceraldehyde-3-phosphate dehydrogenase, amylase, phosphorylase, D-nase, P-nase, and the like. Labeling methods using these labeling substances can be carried out in accordance with known methods (see, for example, "Monoclonal Antibody", Tatsuo Iwasaki et al., Kodansha Scientific Ltd., 1984; and "Enzyme Immunoassay" 2nd edition, Eiji Ishikawa et al., Igaku-Shoin Ltd., 1982).

The measurement of enzyme activity can also be carried out in accordance with known methods depending on the type of enzyme to be used. For example, when peroxidase is used as a labeling enzyme, ABTSJ (2,2'-azino-bi(3'-ethylbenzthiazoline sulfonic acid)) is used as a substrate, or when alkaline phosphatase is used, p-nitrophenyl phosphate is used as a substrate, and the decomposition of each substrate can be measured with a spectrophotometer or the like (see, for example, "Enzyme Immunoassay" 2nd edition, Eiji Ishikawa et al., Igaku-Shoin Ltd., 1982).

When labeling substances of radioactive isotopes, fluorescent substances, and like are used in place of the above labeling enzymes, the measurement of these labeling substances can also be carried out in accordance with known methods.

The solvent used in the above measurement system may be any of generally used solvents that do not adversely affect the reaction. Specifically, buffers having a pH of about 5 to 9, such as citrate buffer, phosphate buffer, Tris salt buffer, and acetate buffer, can be suitably used.

Immune reaction (binding) conditions are also not particularly limited, and usual conditions generally used in this type of assay are employed. In general, the reaction can be carried out at a temperature of 45°C or less, and preferably about 4 to 40°C, for about 1 to 40 hours.

When the measurement target is a kidney biopsy sample, a method using immunostaining can also be employed.

If the amount of anti-spectrin β IgA antibodies determined as described above is larger than the amount of anti-spectrin β IgA antibodies in a healthy subject or a patient with renal disease other than IgA nephropathy, it can be determined that the sample derived from a living organism is related to IgA nephropathy.

Anti-spectrin β IgA antibody measurement reagents, typified by the immunological measurement reagent containing spectrin β, are useful as diagnostic agents for IgA nephropathy.

The anti-spectrin β IgA antibody measurement reagent is generally preferably an immunological measurement reagent containing spectrin β, and more preferably an immunological measurement reagent containing spectrin β-II.

Since anti-spectrin β IgA antibodies are autoantibodies related to IgA nephropathy, drugs which inhibit the activity or production of anti-spectrin β IgA antibodies are clearly useful as preventive or therapeutic agents for IgA nephropathy, and preventive or therapeutic agents for IgA nephropathy can be screened by screening drugs which inhibit the activity or production of anti-spectrin β IgA antibodies.

As the means for screening drugs which inhibit the activity or production of anti-spectrin β IgA antibodies, for example, spectrin β and anti-spectrin β IgA antibodies are reacted in the presence of a test substance, and it is confirmed that the presence of the test substance inhibits the reaction between the antigen and the antibodies. For the reaction between spectrin β and anti-spectrin β IgA antibodies, a reaction system similar to the immunoassay method described above can be used.

Alternatively, screening can be performed by culturing cells which produce anti-spectrin β IgA antibodies in the presence of a test substance, and examining the influence of the addition of the test substance on the production of anti-spectrin β IgA antibodies.

The drug which inhibits the activity or production of anti-spectrin β IgA antibodies selected by such screening is useful as a preventive or therapeutic agent for IgA nephropathy. Specific examples of the drug which inhibits the activity or production of anti-spectrin β IgA antibodies include a chimeric antibody obtained by combining the variable region of an anti-spectrin β IgA antibody with the Fab region of human IgG, and the like.

The preventive or therapeutic agent for IgA nephropathy may contain a drug which inhibits the activity or production of anti-spectrin β IgA antibodies, and may be in the form of a pharmaceutical composition further containing a pharmaceutically acceptable carrier.

The pharmaceutical composition of the present invention can be formed into a composition for oral administration or a composition for parenteral administration. In the case of a composition for oral administration, the pharmaceutical composition of the present invention can be formulated into dosage forms, such as tablets, pills, sugar-coated preparations, soft capsules, hard capsules, solutions, suspensions, emulsions, gels, syrups, and slurries, together with pharmaceutically acceptable solvents, excipients, binders, stabilizers, dispersants, and the like.

In the case of a composition for parenteral administration, the pharmaceutical composition of the present invention can be formulated into dosage forms, such as solutions for injection, suspensions, emulsions, creams, ointments, inhalants, and suppositories, together with pharmaceutically acceptable solvents, excipients, binders, stabilizers, dispersants, and the like. In the case of a composition for injection, it can be dissolved in an aqueous solution, preferably in a physiologically compatible buffer, such as Hank's solution, Ringer's solution, or physiological saline buffer.

### Examples

Next, the present invention will be described in detail with reference to Examples; however, the present invention is not limited thereto.

### Example 1

### (Materials and methods)

### (1) Mice

By selective mating of early-onset ddY mice over 20 generations, gddY mice were established. C57BL/6 NCrSlc, BALB/c, NZB/w F1, and Fas^{lpr/lpr} mice were purchased from Sankyo Labo Service Corporation, Inc. In this test, female NZB/W F1 and Fas^{lpr/lpr} were used.

### (2) Human subjects

With informed consent and approval from the Research Ethics Review Committee of Juntendo University Hospital, the serum from patients with IgA nephropathy or other renal diseases and from volunteers, and kidney biopsy specimens from IgA nephropathy patients were obtained at Juntendo University Hospital.

### (3) Immunofluorescence and electron microscope

Isolated mouse kidneys were fixed in 4% paraformaldehyde for 6 hours, followed by replacement with 30% sucrose at 4°C overnight. The specimens were embedded in OCT compound (Sakura Finetek), frozen in liquid nitrogen, and subjected to immunofluorescence staining. Frozen sections with a thickness of 6 µm were reacted using 3% BSA/PBS for 60 minutes at room temperature to block non-specific staining. Using 10-fold diluted mouse serum as the primary antibody, the kidney sections were stained overnight at 4°C, and reacted with anti-IgA antibody as the secondary antibody for 1 hour. After washing, the slides were mounted with ProLong Gold Antifade Reagent with DAPI (Invitrogen). The anti-IgA antibody used was PE-goat anti-mouse IgA antibody (Abeam). Biotinylated anti-CD138 (BioLegend) and APC-conjugated streptavidin (BioLegend) were used to stain PCs infiltrating the mouse kidneys. All samples were photographed with a confocal microscope (FV3000; Olympus).

After kidney biopsy specimens from the patients were fixed in 15% formaldehyde and embedded in paraffin, sections with a thickness of 3 µm were prepared. After antigen retrieval was performed at room temperature for 2 hours by heat induction for 40 minutes (for CD138 staining) or using 0.05% bacterial protease subtilisin A (Sigma-Aldrich; for IgA staining), immunohistochemical staining was performed. The slides were incubated with the primary antibody for 30 minutes (anti-IgA, Dako Sharp IR51061; anti-CD138, 1:50, Dako #7228), and reacted for 30 minutes using EnVision+ System HRP-Labelled Polymer Anti-mouse (Dako). After that, the color was developed with Liquid DAB+ Substrate Chromogen System (Dako).

### (4) WB method

Balb/c mouse-derived MCs and primary human MCs were used to detect IgA autoantibodies in the serum derived from gddY mice or IgAN patients, respectively. HEK293T cells were infected with vectors expressing full-length or divided mouse Sptbn1 and full-length human SPTBN1, and the resulting cell lysates were used to detect anti-spectrin β IgA antibodies in the serum. WB was performed by a previously reported conventional method. Briefly, cells were lysed with 1% NP-40 lysis buffer containing protease inhibitors. The cell lysates were reduced and denatured with SDS, then boiled and subjected to SDS-PAGE, followed by immunoblotting using the serums and antibodies. Mouse and human serums diluted at 1:40 and 1:50, respectively, were used as the primary antibodies. For the detection antibody, peroxidase-conjugated anti-mouse IgA antibody or anti-human IgA antibody or IgA1 was used.

### (5) Immunoprecipitation

In order to identify the autoantigen recognized by gddY mouse-derived serum IgA, the entire tissue of the kidney or isolated glomeruli derived from C57BL/6 mice was lysed in 1% NP-40 lysis buffer containing protease inhibitors. Balb/c or gddY mouse serum (mixed serum from 10 mice) was reacted with goat polyclonal anti-mouse IgA antibody coupled to NHS-activated Sepharose 4 Fast Flow (GE Healthcare) for 6 hours. Serum IgA was eluted with 0.1 M Glycine-HCl pH 2.0, and the lysate was replaced with PBS by dialysis. Then, the purified serum IgA was directly bound to Pierce NHS-Activated Magnetic Beads (Thermo Fisher Scientific) and used for immunoprecipitation of glomerular proteins recognized by serum IgA.

### (6) Mass spectrometry

The glomerular proteins immunoprecipitated with the Balb/c or gddY mouse serum IgA were separated by SDS-PAGE. Portions of the gel containing the protein precipitated only by the gddY mouse serum IgA were excised and destained. Then, the gel pieces were dehydrated with 100% acetonitrile (ACN) at room temperature for 10 minutes, and dried under vacuum. Next, the gel pieces were reduced simultaneously with 10 mM Tris(2-carboxyethyl)phosphine hydrochloride (Thermo Fisher) and 40 mM chloroacetamide (Sigma) in 100 mM Tris-HCl (pH: 8.5), alkylated, and incubated at 70°C for 5 minutes. After reduction/alkylation, the solution was removed, and the gel pieces were each washed with 50% ACN for 10 minutes on Intelli-Mixer RM-2M (ELMI Ltd., Latvia) with shaking at room temperature. After that, the gel pieces were dehydrated with 100% ACN for 10 minutes and dried. 20 ng/L trypsin/Lys-C (Promega) in 100 mM HEPES (pH: 8.5) was added to each gel piece and kept on ice for 30 minutes. The gel pieces were then incubated at 37°C overnight. After digestion, peptides were extracted three times from the gel pieces with 0.1% formic acid in 50% ACN, and the solutions were collected in the same vial each time. The resulting peptides were desalted using SDB tips (GL Science, Tokyo) and lyophilized using a centrifugal evaporator EZ-2 Elite (Genevac Ltd., Ispswich, UK).

Proteins were identified by nano-LC-MS/MS using a Triple TOF 5600+ system and an Eksigent nano LC system (AB Sciex, MA) operated with Analyst TF 1.7 software. To the software (AB Sciex) protein identification using the UniProt database (2020_04) which searched the obtained MS data with ProteinPilot 5.0.1, a confidence cutoff of 1% false discovery rate was applied.

### (7) Plasmid construct

In order to produce a mouse full-length or divided Sptbn1 expression vector, cDNAs were prepared from isolated glomeruli of C57BL/6 mice. From these cDNAs, the full-length sequence of sptbn1 was amplified using KOD plus Neo DNA Polymerase (Toyobo) and subcloned into pCAT7-neo vector (a vector expressing a double T7-labeled protein at the N-terminus of the target protein). As for mouse divided sptbn1, the sequences of sptbn1A (CDS 4..2364>), sptbn1B (CDS 2365..4728>), and sptbn1C (CDS 4729..7092) were amplified using KOD Fx Neo or plus Neo DNA Polymerase (Toyobo), and subcloned into pCAT7-neo vector or p3XFLAG-CM-10 (Sigma-Aldrich). A FLAG-tagged human full-length SPTBN1 expression vector was provided by Dr. Imamichi (J Exp Med. 2013; 210 (3): 517-34).

For HA-tagged I-Aα and FLAG-tagged I-Aβ derived from gddY or C57BL/6 mice, cDNAs were prepared from isolated naive B cells of gddY or C57BL/6 mice. From these cDNAs, the sequences of I-Aα and I-Aβ were amplified using KOD plus Neo DNA Polymerase (Toyobo), and subcloned into a pMX vector. A HA- or FLAG-tag sequence and a linker peptide sequence (aggtggaggcagc) were added to the N-terminus of I-Aα or I-Aβ.

### (8) ELISA assay

In accordance with the method described in Kidney Int. 2007; 72 (3): 319-27, total serum IgA were measured by sandwich ELISA.

### (9) Tissue preparation, flow cytometry, and single B cell sorting

Perfused kidneys were cut into 2- to 3-mm pieces and destroyed with 0.6 mg/mL collagenase D (Roche) and 100 ug/mL DNase I (Roche). Then, mononuclear cells of the kidney were isolated by a gradient centrifugation method using Percoll. Mononuclear cells of the small intestinal lamina propria of gddY mice were isolated as described in Nature Communications 2019; 10 (1): 3650. The single cell suspension was stained with the following reagents. BD Bioscience: B220 (APC-Cy7), CD138 (PE or BV421), GL7 (PerCP Cy5.5), Ki67 (PE-Cy7), IgG1, G2a, G2b, G2c, and G3 (FITC), IgA (biotin), and streptavidin (VB421 or APC).

IgA PBs and IgG PCs were stained by intracellular staining using Fixation and Permeabilization Solution Kit (BD Biosciences). Ki67 was stained by intracellular staining using Foxp3 Staining Buffer Set (eBioscience).

To isolate single IgA PBs from the kidney of gddY mice, mononuclear cells isolated from the gddY kidney were surface-stained with CD138 (PE), IgA (biotin), and streptavidin (APC). Single CD138+ IgA+ PBs were isolated for each cell in a 96-well PCR plate (Eppendorf) containing 4 uL of lysis solution. The surface expression of MC I-A was confirmed by using anti I-A antibodies (PE, Southern Biotech). All samples were analyzed using FACS Calibur, FACS Aria II, or FACS Canto II (BD Biosciences). The data were analyzed using FlowJo (Tree Star).

### (10) Cell culture

IgA+ PBs derived from the kidney or small intestine of gddY mice were co-cultured with feeder cells expressing CD40L, a B-cell activating factor, and a proliferation-inducing ligand in "B-cell medium" (RPMI-1640 medium (Wako)) supplemented with interleukin-6 (IL-6), 10% fetal bovine serum (FBS), 10 mM HEPES, 1 mM sodium pyruvate, 5.5×10-5 M 2-ME, 100 U/mL penicillin, and 100 µg/mL streptomycin (Gibco) in an incubator with 5% CO₂ at 37°C. The culture supernatant of IgA+ PBs was collected on the 6th day.

MCs isolated from Balb/c mice were used. MCs were cultured in Dulbecco's modified Eagle's medium (high glucose) containing L-glutamine, phenol red, and sodium pyruvate (DMEM) supplemented with 10% FBS, 10 mM HEPES, 100 U/mL penicillin, and 100 ug/mL streptomycin. MCs were used between passages from 8 to 16.

### (11) preparation of recombinant antibodies ADDIN EN. CITE ADDIN EN. CITE. DATA

Recombinant antibodies from IgA PBs in the kidney of gddY mice were generated as described in J Immunol Methods. 2009; 350 (1-2): 183-93. On an original 96-well plate with nuclease-free water (Eppendorf) (final volume: 20 uL/well) using SuperPrep II Cell Lysis & RT Kit (Toyobo), the total RNA extracted from IgA+ PBs isolated from the kidney of gddY mice was subjected to reverse transcription (RT) reaction to synthesize cDNA. The RT reaction was carried out at 42°C for 5 minutes, 25°C for 10 minutes, 50°C for 60 minutes, and 94°C for 5 minutes, and the cDNA was stored at -20°C until use. The IgA heavy chain gene and the Igκ or Igλ light chain gene were amplified by nested PCR (at 98°C for 10 seconds, 55°C for 30 seconds, and 68°C for 30 seconds, 40 cycles). After identification of IgV and J genes by IgBlast (http://www.ncbi.nlm.nih.gov/igblast/) and VBASE2 (http://www.vbase2.org/), 0.5 uL of unpurified first-round PCR product was used as a template, and the second PCR reaction was carried out using single gene-specific V- and J-gene primers containing restriction sites (variable region PCR product). The number of somatic mutations in the V gene was counted, including FWR1 to FWR3. The variable region PCR product and the pCAGGS expression vectors containing the mouse IgA, Igκ, or Igλ constant region were treated with restriction enzymes and then ligated using Ligation high Ver. 2 (Toyobo). This plasmid was transformed into competent *E. coli* HST08 bacteria via heat shock at 42°C and the resulting bacteria was cultured on ampicillin plates (100 µg/mL). After purifying the expression vector from the colonies, the PCR product was sequenced to confirm its identity with the second round PCR product. HEK293T cells were transiently transfected with vectors expressing heavy and light chains each in an equal amount of 1 µg. Three days after transfection, the supernatant was collected, the medium was replaced with fresh culture medium, and the culture supernatant was collected again 6 days after transfection. The culture supernatant was removed from cell debris by centrifugation at 1400 rpm for 10 minutes, and the resultant was stored at -20°C until use. As a negative control, a recombinant IgA antibody against NP with VH 186.2, DFL 16.1, JH 2 in the heavy chain, and λ1 in the light chain was produced.

### (Results)

### (1) IgA autoantibodies against mesangial cells are present in the serum of gddY mice.

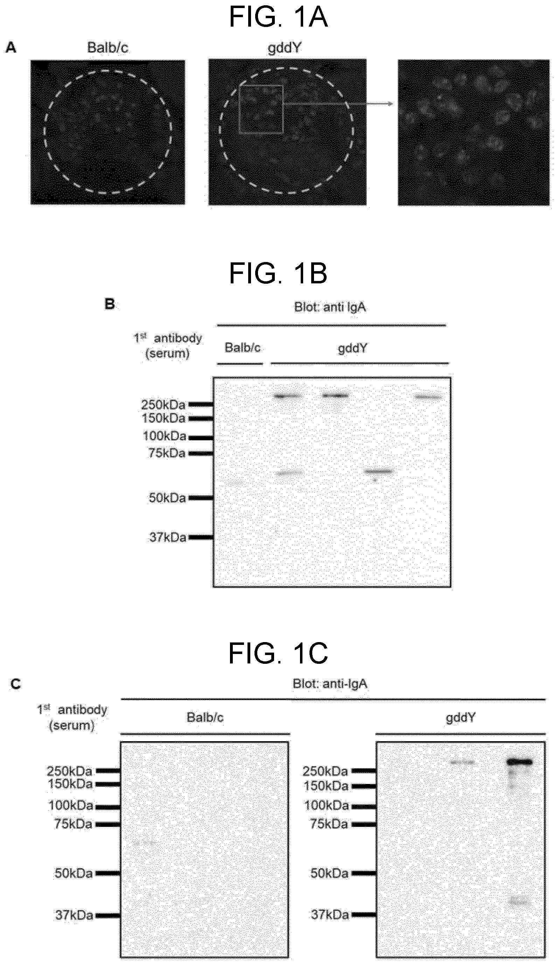

Antibody deposition in different tissues suggests antigen recognition by autoantibodies. Therefore, the possibility that IgA autoantibodies against glomerular MCs were present in the serum of gddY mice was examined. First, kidney sections of AID-deficient mice lacking endogenous IgA antibodies were immunofluorescence-stained with the serum of Balb/c or gddY mice as the primary antibody, and then with anti-mouse IgA as the secondary antibody. As a result, only gddY mouse-derived serum IgA bound to the glomeruli, whereas Balb/c mouse-derived serum IgA did not (Figure 1A), suggesting that IgA antibodies against some of the antigens expressed in the renal glomeruli were present in the gddY mouse serum. Next, this result was confirmed by WB using glomerular proteins isolated from the gddY mouse kidney. Serum IgA derived from gddY mice bound to some of the glomerular proteins, among which IgA antibodies against a protein with a molecular weight of approximately 250 kDa (referred to as p250+) were frequently detected (Figure 1B) .

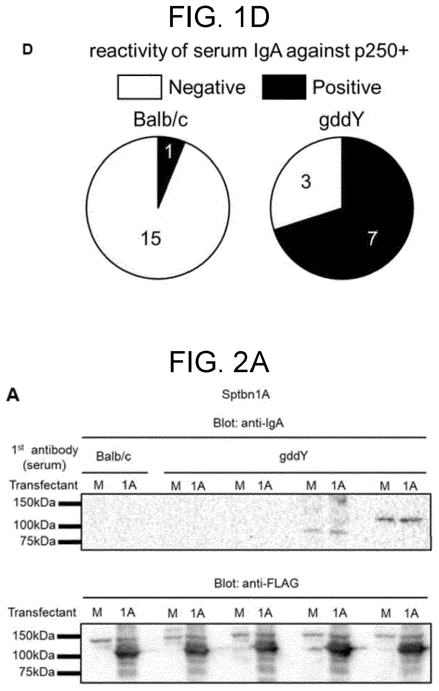

Next, since IgA molecules are deposited in the mesangial region of renal glomeruli in IgA nephropathy, WB was performed using primary cultured MCs derived from the kidney of Balb/c mice. As a result, gddY mouse-derived serum IgA significantly detected the band of p250+ (Figure 1C), revealing that p250+ is the major target antigen of IgA autoantibodies in the gddY mouse serum. Seven out of 10 gddY mice in total had serum IgA recognizing p250+, whereas only 1 out of 16 Balb/c mice had such serum IgA (Figure 1D).

### (2) Spectrin β is a target antigen of IgA autoantibodies in gddY mice.

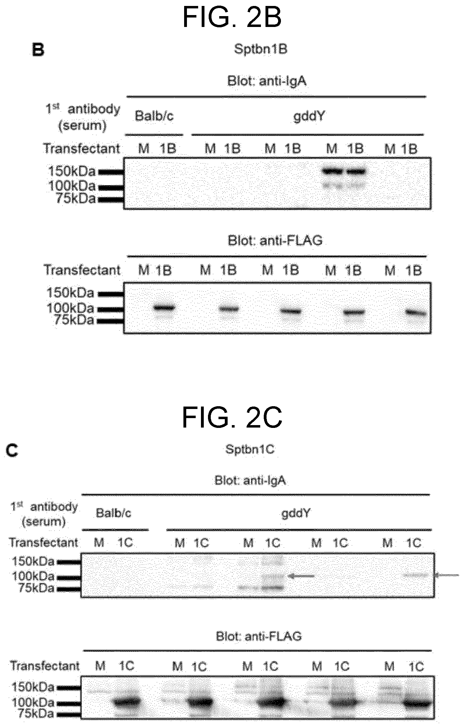

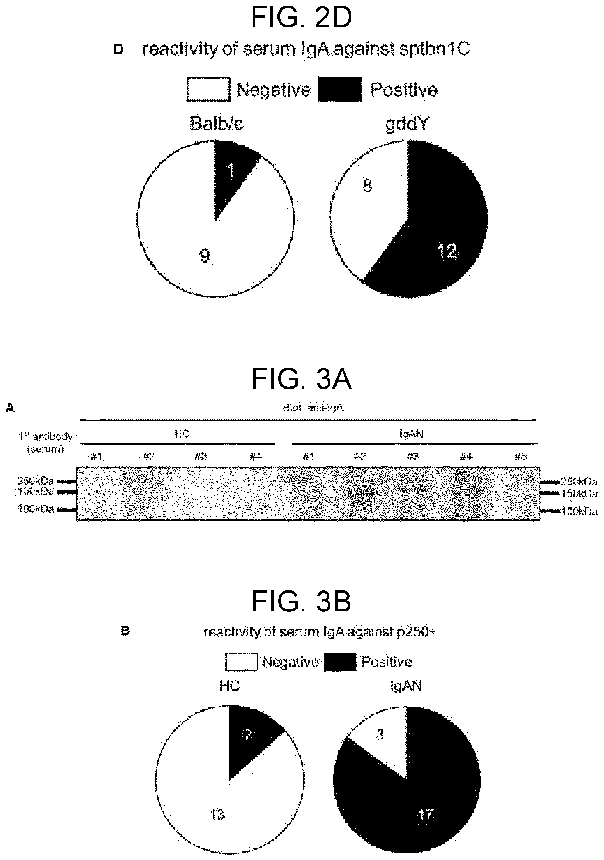

In order to identify the autoantigen recognized by gddY mouse serum IgA autoantibodies, immunoprecipitation was performed on mouse renal glomerular proteins using the serum of gddY mice or Balb/c mice. The precipitated proteins were separated by SDS-PAGE, and proteins precipitated only by the gddY mouse serum were analyzed by mass spectrometry. Expression vectors of the candidate autoantigens identified were prepared and infected into HEK293T cells. Autoantigens were screened by WB using this cell lysate and gddY serum. As a result, spectrin β chain nonerythrocytic 1 (referred to as Sptbn1) was identified as the protein recognized only by gddY mouse serum IgA. Next, the region recognized by the autoantibodies was narrowed down. Then, Sptbn1 was divided into 3 regions (Sptbn1A: 1-788aa; Sptbn1B: 789-1576aa; and Sptbn1C: 577-2364aa), each fragment was separately expressed in HEK293T cells, and WB was performed. Among them, the Sptbn1C fragment was specifically recognized by gddY mouse serum IgA (Figures 2B to D). From this assay, 12 out of 20 gddY mice had a total of serum IgA autoantibodies against Sptbn1C (Figure 2E) .

Among other candidate proteins (10 or more) tested in the same WB assay, only the C-terminal fragment of spectrin α-chain nonerythrocytic 1 (referred to as Sptan1C) was selectively recognized by gddY mouse serum IgA. However, the positive frequency of anti-Sptan1C IgA antibodies in gddY mice was much lower than that of anti-Sptbn1 IgA antibodies (3 out of 22 mice), suggesting that Sptbn1 was a major target antigen of IgA autoantibodies in gddY mice.

### (3) IgA nephropathy patients possess IgA autoantibodies against antigens expressed in mesangial cells.

The possibility that IgA autoantibodies against MCs were present in the serum of IgA nephropathy patients was examined by WB using proteins extracted from human MC cells. As a result, the serum of most IgA nephropathy patients contained IgA recognizing p250+ (85%). On the other hand, the IgA antibody-positive rate in the serum of healthy subjects was as low as 13% (Figures 3A and B).

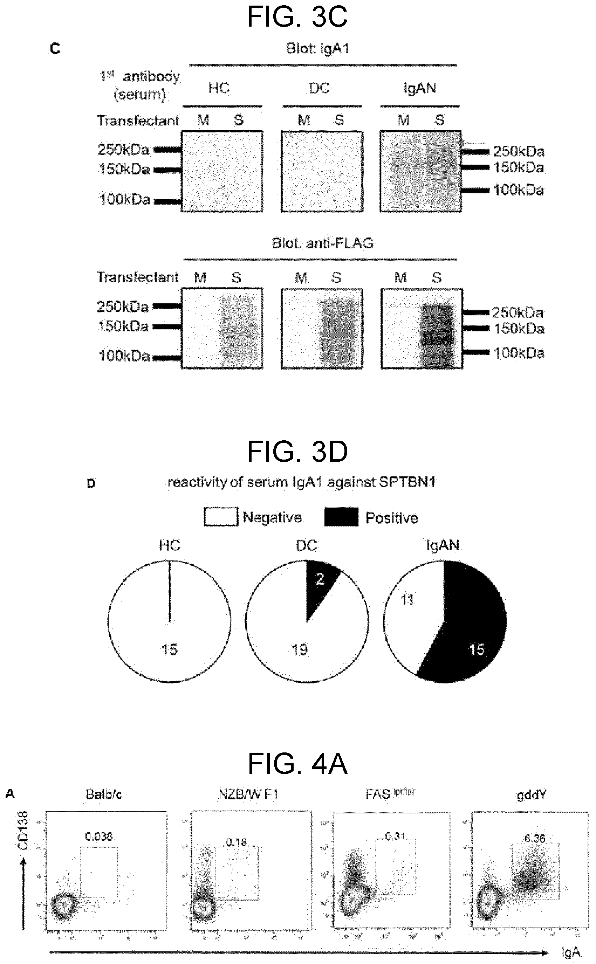

Based on the similarity in size of p250+ proteins recognized by the serum derived from IgA nephropathy patients and gddY mice, it was hypothesized that serum IgA of IgA nephropathy patients recognized human SPTBN1. Then, WB was performed using HEK293T cells infected with a mock vector or a vector expressing human SPTBN1 (full length) and patient serum. As a result, 15 out of 26 IgA nephropathy patients possessed anti-SPTBN1 IgA1 antibodies. In contrast, no serum anti-SPTBN1 IgA1 antibodies were observed in the serum of healthy subjects, and in other renal disease patients, only 2 of 21 cases were positive and less frequent (Figures 3C and 3D). These results clarified the presence of IgA1 autoantibodies against SPTBN1 specific to IgA nephropathy patients.

### (4) IgA-positive plasmablasts are accumulated in the kidney of gddY mice.

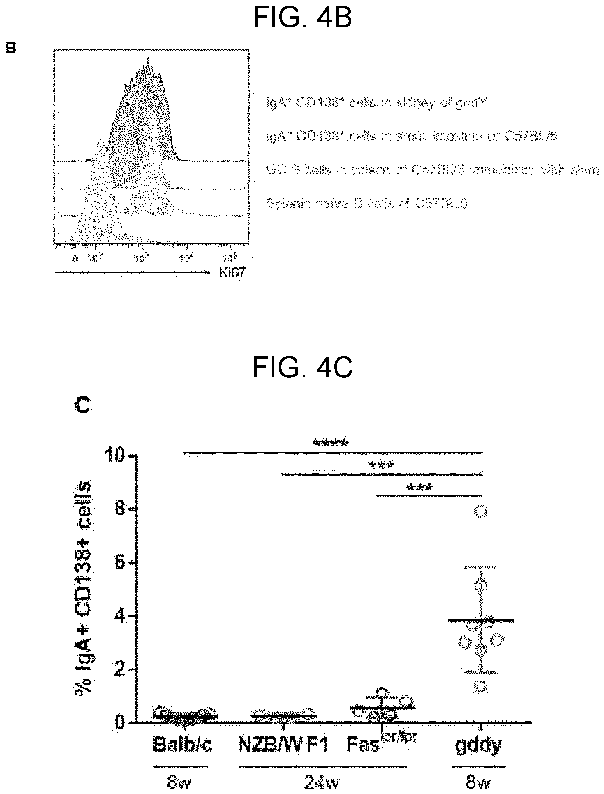

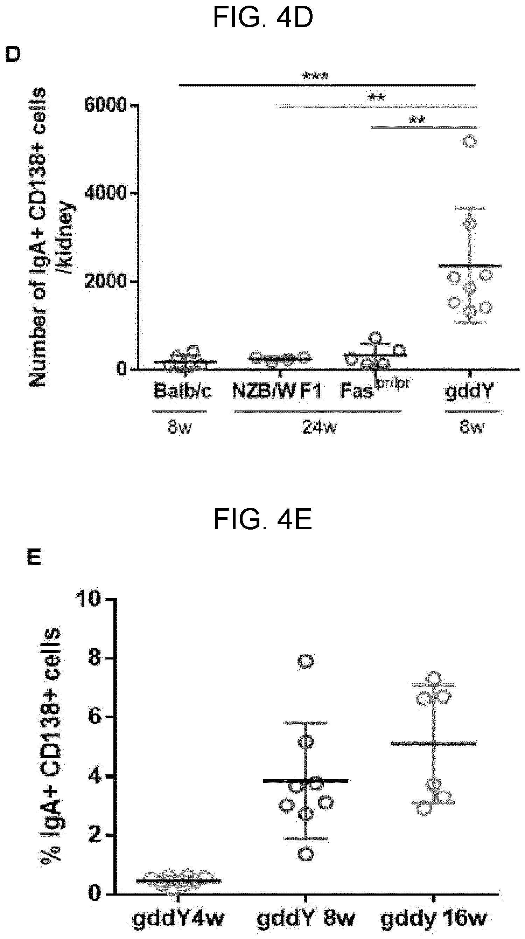

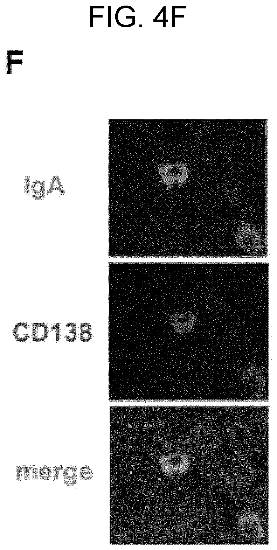

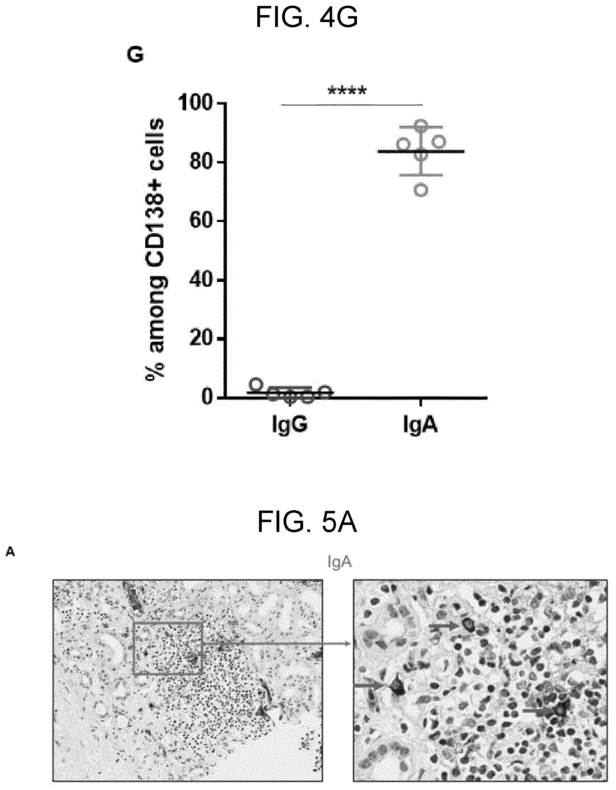

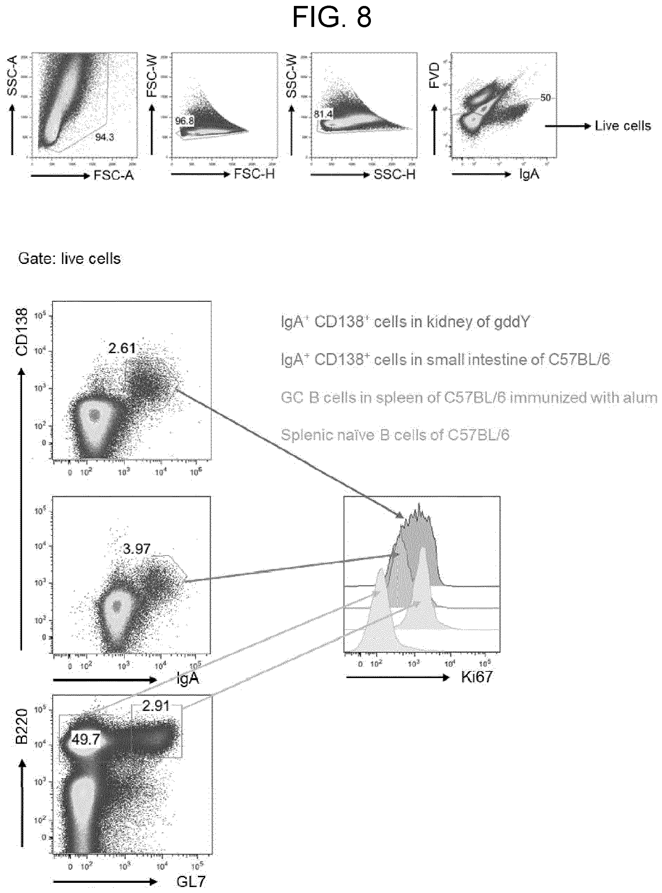

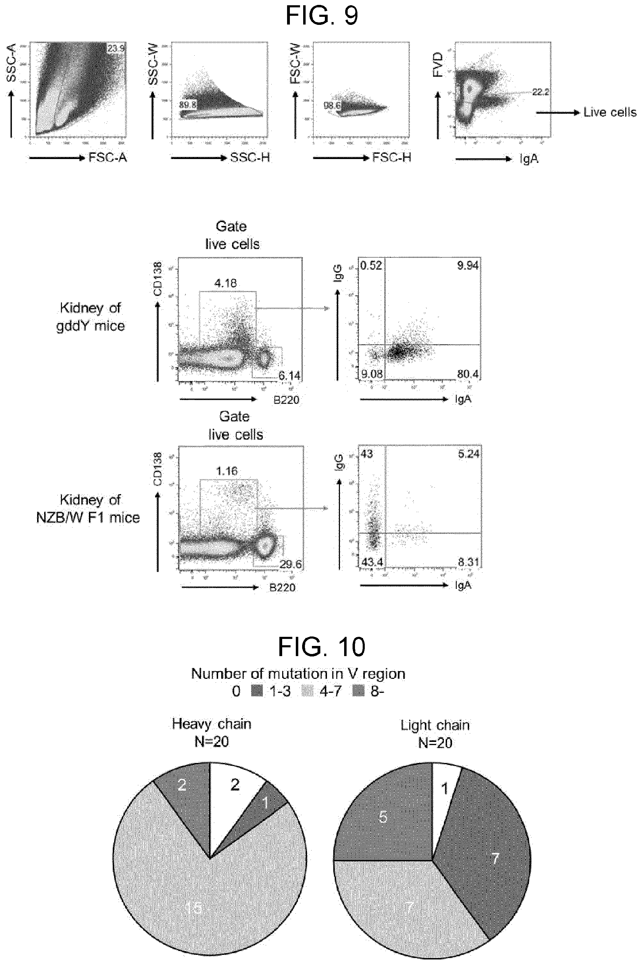

The above results strongly suggest that IgA nephropathy is an autoimmune disease with tissue-specific autoantibodies. In recent years, it has been reported that plasma cells secreting autoantibodies are present in inflamed tissues in some autoimmune diseases. Based on this report, it was hypothesized that the gddY kidney was infiltrated with plasma cells producing autoantibodies. Then, mononuclear cells isolated from the kidney of gddY mice were analyzed by flow cytometry. Balb/c mice and lupus model mice (NZB/w F1 and Fas^{lpr/lpr}) were used as controls. As a result, it was revealed that the gddY mouse kidney was significantly infiltrated with IgA+ CD138+ plasma cells, as compared with other mice (Figures 4A, C, and D). These IgA+ CD138+ cells were positive to Ki67, which is a proliferation marker, as with germinal center B cells (Figures 4B and 8), suggesting that they were short-lived plasmablasts (PBs). The frequency of IgA+ PBs was gradually increased with age in gddY mice (Figure 4E). Immunofluorescent staining of the kidney of 8w gddY mice revealed the presence of IgA+ PBs in the tubulointerstitium (Figure 4F). As previously reported, the subclass of plasma cells infiltrating the kidney of NZB/w F1 mice was dominated by IgG (Figure 9), whereas the subclass of most of plasmablasts of the kidney of gddY mice was IgA (Figure 4G).

### (5) IgA secreting cells are present in the kidney of IgA nephropathy patients.

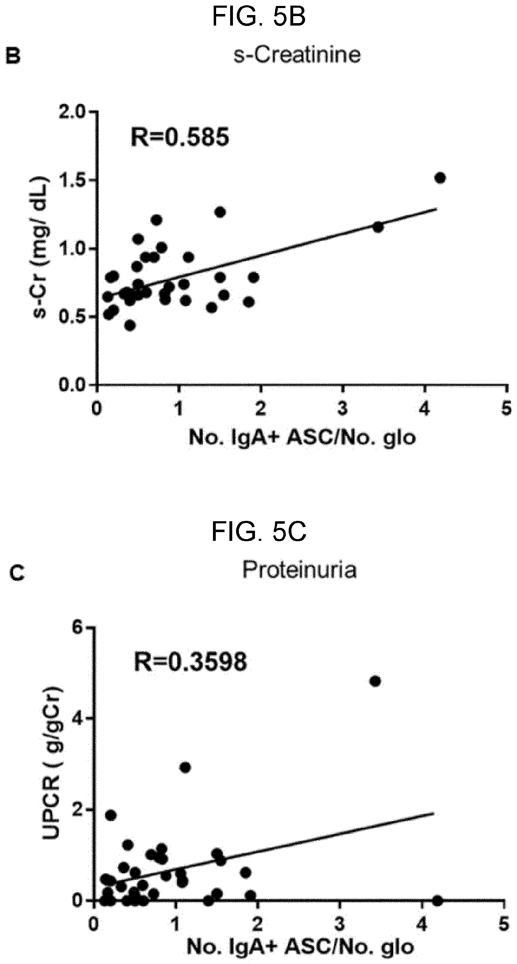

Next, it was confirmed whether IgA antibody secreting cells (ASCs) were also present in the kidney of IgA nephropathy patients. As a result of immunochemical staining of kidney biopsy sections of IgA patients, IgA-positive ASCs were detected in the tubulointerstitial region of the kidney, as in the case of gddY mice (Figure 5A). As reported in SLE patients, the number of infiltrated ASCs correlated with serum creatinine levels and the amount of proteinuria in IgA nephropathy patients (Figures 5B and C).

### (6) IgA+ PBs infiltrating the kidney of gddY mice produce IgA autoantibodies.

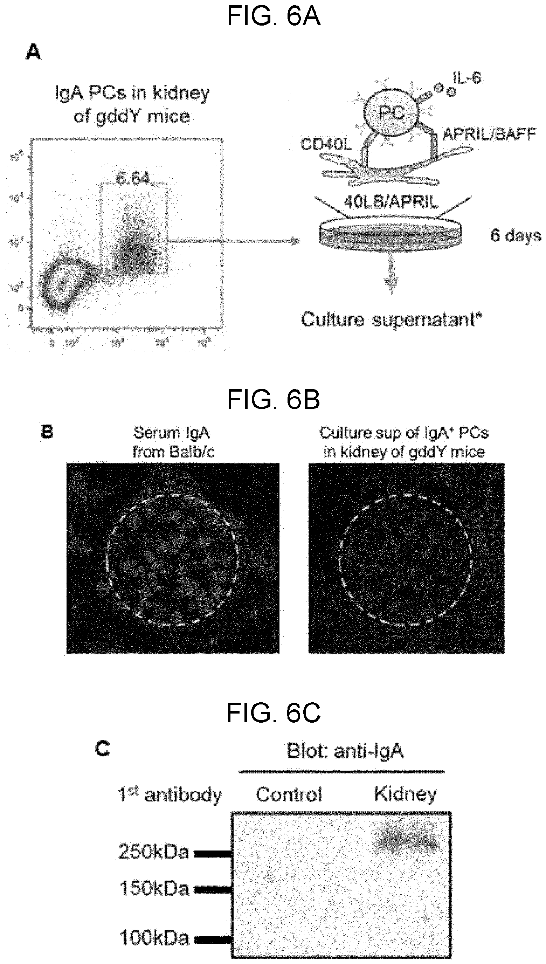

Next, the possibility that IgA+ PBs infiltrating the kidney of gddY mice produce IgA autoantibodies was examined. The cells isolated from the gddY kidney were cultured (Figure 6A) and fluorescent immunostaining and WB were performed using these culture supernatants. As a result, it was revealed that IgA in the culture supernatant bound to glomerular components, particularly p250+ protein (Figures 6B and C). Further, it was also confirmed that these IgAs recognized Sptbn1C (Figure 6D).

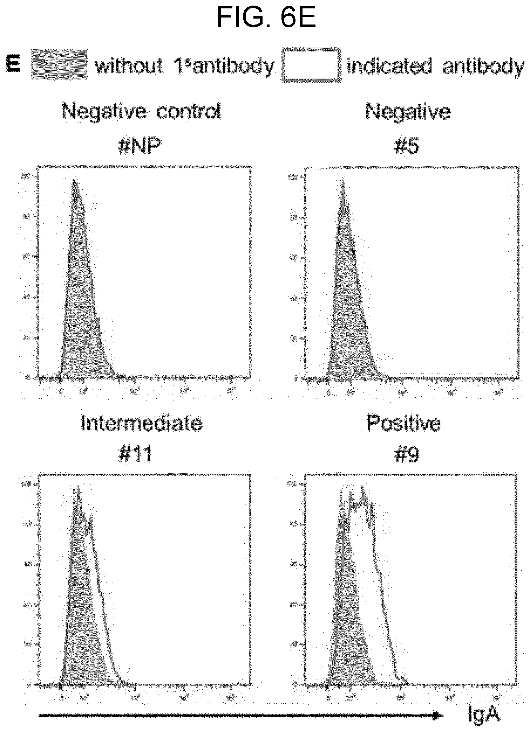

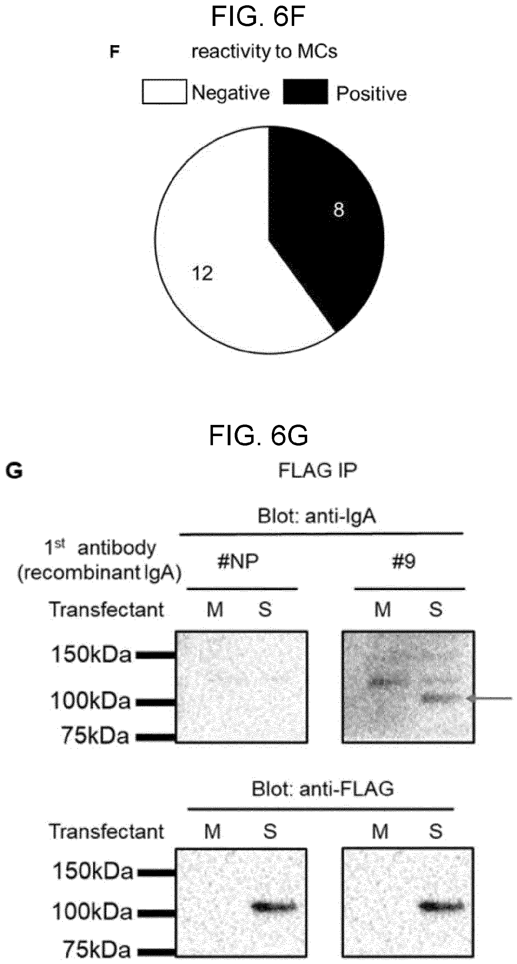

In order to confirm that IgA+ PBs in the kidney of gddY mice produce IgA autoantibodies against antigens in MCs, single IgA+ PBs were isolated from the kidney of gddY mice, and their heavy and light chain genes were cloned to produce recombinant IgA antibodies. Most of the H and L chains contained a considerable number of mutations in their amino acid sequences, which suggests that PBs had undergone somatic hypermutation (Figure 10). Using these recombinant antibodies as the primary antibodies, intracellular staining of MCs was performed, followed by analysis by flow cytometry. As a result, it was revealed that although the intensity of staining differed among the antibodies, 8 out of 20 recombinant antibodies recognized antigens present in MCs (Figures 6E and F) .

Further, it was confirmed by WB that 5 recombinants out of 20 recombinant antibodies recognized Sptbn1C (Figures 6g and H). These results revealed that as with other animal models and autoimmune disease patients, gddY mice had pathological characteristic of autoimmune diseases: infiltration of autoantibody-producing ASCs into inflamed tissue.

### (7) Sptbn1, which is an intracellular protein, is presented on the cell surface by MHC class II molecules.

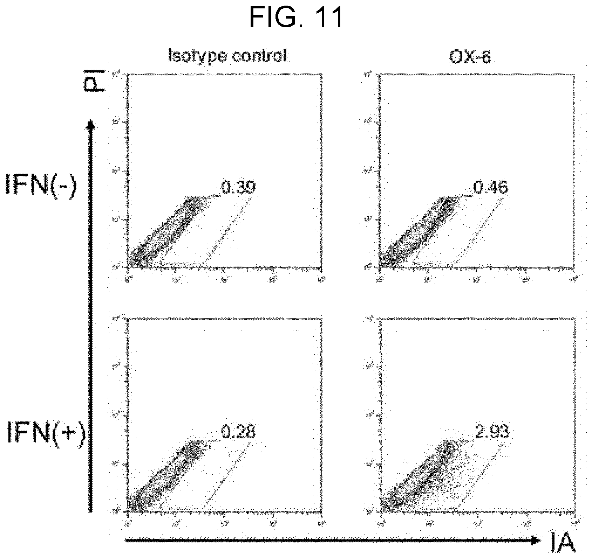

Next, the mechanism by which Sptbn1, an intracellular protein, is recognized by anti-spectrin β antibodies in the serum of gddY mice was explored. In recent years, it has been reported that a misfolded intracellular protein can be transported to the cell surface by binding to MHC class II molecules abnormally expressed in tissue cells and can be a specific target for autoantibodies. MCs are known to express MHC class II both *in vivo* and *in vitro.* In fact, it has been also reported that MHC class II is expressed in renal glomeruli of IgA nephropathy patients. Based on this report, we hypothesized that intracellular Sptbn1 was presented on the surface of MCs by binding to MHC class II molecules. First, it was confirmed by flow cytometry that MHC class II (I-A) was expressed on the gddY mouse-derived MC surface stimulated with IFN-γ (Figure 11). Next, the binding ability of recombinant antibodies prepared from gddY kidney IgA+ PBs to the IFN-γ-stimulated MC surface was analyzed by flow cytometry. As a result, it was revealed by WB that recombinant antibody clone #9 recognizing the MC antigen (Figure Sptbn1C (Figure 6G) bound to the IA-positive MC surface (Figure 7A) .

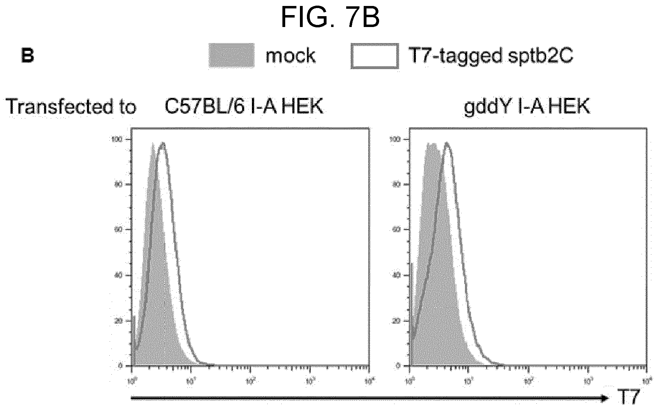

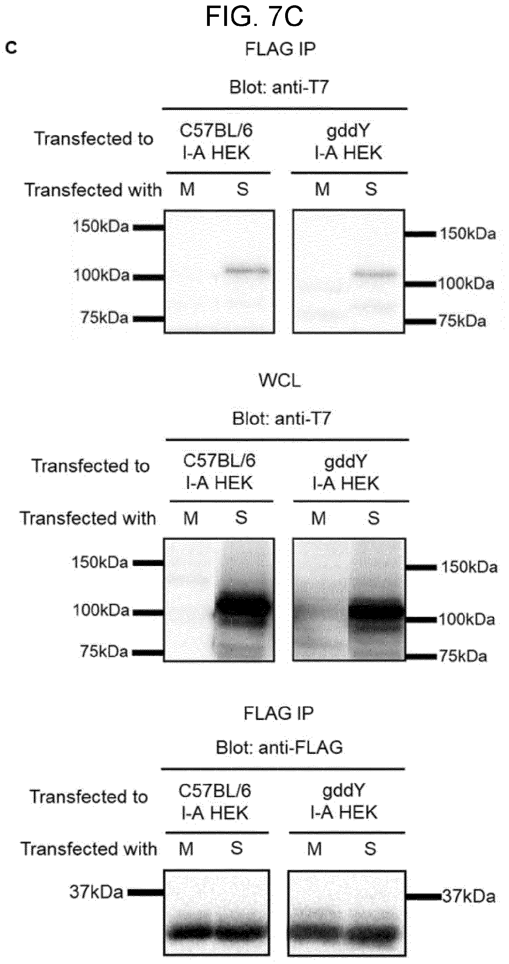

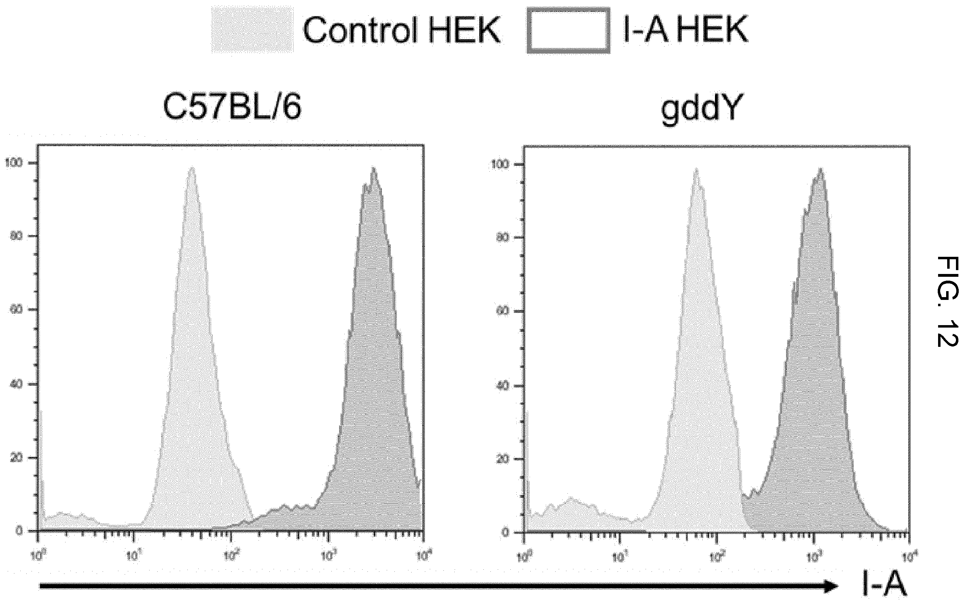

Next, in order to examine whether Sptbn1 can be presented on the cell surface by MHC class II, vectors expressing gddY (H-2S)- or C57BL/6 (H-2b)-derived HA-tagged α-chain and FLAG-tagged β-chain were infected into HEK293T cells using a retrovirus, and a HEK293T cell line (I-A HEK) constitutively expressing I-A was established. The expression of I-A on the I-A HEK cell surface was confirmed by flow cytometry (Figure 12). Next, a T7-tagged Sptbn1C expression vector was overexpressed in I-A HEK, and its surface expression was analyzed by flow cytometry. As a result, it was revealed that Sptbn1C was presented on the cell surface of I-A HEK, regardless of I-A haplotype (Figure 7B). These results were confirmed by analysis using WB. I-A HEK was infected with T7-tagged Sptbn1C, and MHC class II proteins were immunoprecipitated from these cell lysates using anti-FLAG antibodies. Immunoblotting of the precipitated proteins with anti-T7 antibodies revealed that Sptbn1C was co-precipitated with I-A molecules (Figure 7C), and it was confirmed that Sptbn1C associated with I-A molecules.

From the above results, Sptbn1C, which is an intracellular protein, bound to MHC class II molecules to be thereby presented on the MC surface, suggesting that it was recognized by anti-spectrin β IgA antibodies in the serum of gddY mice.

## Claims

1. A method for measuring autoantibodies related to IgA nephropathy, the method comprising measuring amount of anti-spectrin β IgA antibodies in a sample derived from a living organism.

2. A diagnostic agent for IgA nephropathy comprising an anti-spectrin β IgA antibody measurement reagent.

3. The diagnostic agent for IgA nephropathy according to claim 2, wherein the anti-spectrin β IgA antibody measurement reagent is an immunological measurement reagent comprising spectrin β.

4. A method for screening a preventive or therapeutic agent for IgA nephropathy, the method comprising screening a drug which inhibits activity or production of anti-spectrin β IgA antibodies.

5. A preventive or therapeutic agent for IgA nephropathy comprising a drug which inhibits activity or production of anti-spectrin β IgA antibodies as an active ingredient.

6. A drug which inhibits activity or production of anti-spectrin β IgA antibodies for use in prevention or treatment of IgA nephropathy.

7. Use of a drug which inhibits activity or production of anti-spectrin β IgA antibodies for producing a preventive or therapeutic agent for IgA nephropathy.

8. A method for preventing or treating IgA nephropathy, the method comprising administering an effective amount of a drug which inhibits the activity or production of anti-spectrin β IgA antibodies.
